(19)

Europäisches
Patentamt
European
Patent Office
Office européen
des brevets

(11) **EP 2 600 264 A1**

(12) **EUROPEAN PATENT APPLICATION**

(43) Date of publication:
**05.06.2013 Bulletin 2013/23**

(51) Int Cl.:
*G06F 19/00* $^{(2011.01)}$

(21) Application number: **11191630.0**

(22) Date of filing: **01.12.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Fujitsu Limited**
**Kawasaki-shi, Kanagawa 211-8588 (JP)**

(72) Inventors:
• **Naseer, Aisha**
 **Hayes, Middlesex UB4 8FE (GB)**

• **Hu, Bo**
 **Hayes, Middlesex UB4 8FE (GB)**
• **Fukuda, Kenichi**
 **Hayes, Middlesex UB4 8FE (GB)**

(74) Representative: **Wilding, Frances Ward**
 **Haseltine Lake LLP**
 **Lincoln House, 5th Floor**
 **300 High Holborn**
 **London WC1V 7JH (GB)**

Remarks:
Amended claims in accordance with Rule 137(2) EPC.

(54) **A computer-implemented healthcare system and method**

(57)  Embodiments of the invention provide a computer-implemented healthcare system arranged to produce personal advices for a user, the system comprising: a goal analyser arranged to receive a healthcare goal, to receive personal risk factors for a user and to provide atomic goals from a combination of the personal risk factors and the healthcare goal; an engine finder arranged to input atomic goals to one or more selected advice generation engines, which provide advices relating to the atomic goals; and an advice filter, which filters the advices from the selected reasoning engines against checking parameters to provide checked advice for output.

FIG. 3

## EP 2 600 264 A1

### Description

The present invention relates to a computerised healthcare advice system and method. It has industrial application in the healthcare industry, in preventative, curative and palliative care.

### Background

Prevention could save tens of thousands of lives each year and improving control is one of the most effective ways to prevent chronic conditions such as heart disease, diabetes, or stroke. Since lifestyle interventions could modify the risk factors therefore awareness about the available lifestyle options is imperative for self-management.

In the EU, there is an increase of €299 billion per year in the total medical cost, which could be parsed into: €80 billion for obesity; €110 billion for diabetes; and €109 billion for cardiovascular diseases. It has been reported that NHS spends 75% of its budget for treating Long Term Conditions (LTCs) which could be prevented by self-management and adopting healthy lifestyle options.

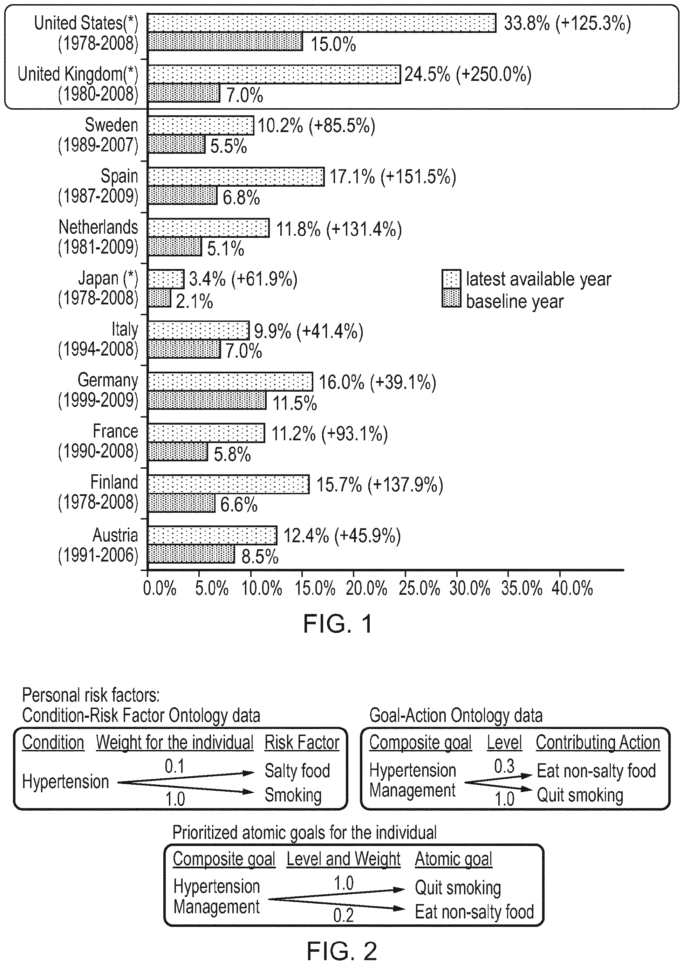

Fig 1 shows the prevalence of obesity (i.e. BMI $\geq$ 30) in adult population aged 20-79 years with US and UK as the top two countries bearing highest obesity levels. According to a study 75% of the risk of type-II diabetes is attributable towards obesity, which is preventable through self-awareness and self-management (i.e. by changing lifestyles. Moreover, 80% of all cardiovascular diseases are considered to be preventable by reducing risk factors like smoking and unhealthy diet. According to the World Health Organisation (WHO) at least 80% of all cases of heart disease, stroke and diabetes are preventable requiring lifestyle changes.

It has been reported that in order to shape future healthcare, more effective preventive measures and fundamental lifestyle changes need to be promoted to encourage healthy behaviour. The report by Economist Intelligence Unit also identifies five extreme scenarios for European healthcare in 2030, one of which is the precedence of preventive care over treatment of the ill.

One of the key steps towards preventive care is the adoption of healthy and active lifestyle and thus prevalence of chronic diseases could be prevented by promoting healthy lifestyle information to not only the individuals who are at risk but also to those who might become at risk in the near future due to unhealthy lifestyles. Technology can help chronic disease prevention by disseminating information to patients and clinicians, and enabling effective surveillance of chronic conditions to guide policy development and interventions.

Individuals can benefit from increased awareness of the consequences of risk factors, as well as access to resources and guidance specifically relating to their chronic condition, which can be especially beneficial in preventing co-morbidities [Public Health Agency of Canada, 2009]. Moreover, Governments could benefit economically from their focus on disease prevention and promotion of public health.

It is an aim of the embodiments of the present invention to provide a resource which can be applied on an individual basis to users (that is patients, or other individuals who receive healthcare recommendations).

The invention is defined in the independent claims, to which reference should now be made. Advantageous embodiments are set out in the sub claims.

According to one embodiment of an aspect of the invention there is provided a computer-implemented healthcare system (arranged to produce personal advices for a user), the system comprising a goal analyser arranged to receive a healthcare goal, to receive personal risk factors for a user and to provide atomic goals from a combination of the personal risk factors and the healthcare goal; an engine finder arranged to input atomic goals to one or more selected advice generation engines, which provide advices relating to the atomic goals; and an advice filter, which filters the advices from the selected reasoning engines against checking parameters to provide checked advice for output.

Furthermore, embodiments of the invention may provide one only of the goal analyser, engine finder and advice filter, or any combination thereof.

Embodiments of the invention provide a method and system that can generate and modify dynamic, context-aware personalised advices also taking into account an individual's situation.

Such embodiments can allow advice for a medical goal, which is input directly, for example by a healthcare advisor. Appropriate goals include, for example hypertension management, weight loss or another such goal. Alternatively, input by a user/healthcare advisor may be a condition to be managed/illness etc and the goal implicitly recognised as improvement or maintenance of the condition/illness.

The starting goal or other input may thus be manually input (by a human advisor) or read-in from a storage device). The personal risk factors can be stored in a domain knowledge base (which is described in more detail later) or may also be input by a human advisor or the user. Most healthcare goals can be seen as comprising multiple sub-goals, thus the atomic goals which are provided are essentially based on goal decomposition of the main healthcare goal.

The engine finder takes these atomic goals and uses them to select one or more advice generation engines, which may be provided within the computer-implemented healthcare system or may be external engines. These engines may be essentially reasoning engines such as inference engines which generate advices. They are built in software or hardware or a combination of the two. The engine finder may select one or more engines per atomic goal. The advice filter checks and optionally modifies the advices produced so that the output advice has been fully processed. The checking parameters can include advice compatibility with other advices, soft and/or hard constraints, personal preferences or any other suitable parameter.

These system components allow provision of advice which is appropriate to the user and therefore more likely to gain acceptance and adoption. Decomposition of the main goal using risk factors allows such personalisation and gives a detailed advice set.

The goal analyser of invention embodiments may be arranged in any suitable fashion for its purpose of the provision of atomic goals. Preferably, the goal analyser is arranged to analyse the healthcare goal and the personal risk factors in conjunction with a goal-action ontology, and produce a list of actions, which is preferably weighted according to the level of contribution of the action on the personal risk factors and the significance of the personal risk factors for the user; and preferably to select actions having a weight value above a threshold as atomic goals.

This use of an ontology, which may be stored in the domain knowledge base mentioned above or created on the fly, allows a language-based, easily updatable decomposition into atomic goals.

Thus the goal analyser is arranged to use text analysis, preferably in the form of NLP (Natural Language Processing) techniques. For simpler implementations, the atomic goals need not be weighted.

In the further optional operation, any goal analyser may compute priority for each atomic goal by association with universal risk factors for poor health, such as smoking, high blood pressure, alcohol, over-weight etc.

The actions may further be selected if they have a weight value above a threshold, to allow less important actions to be discarded.

Moreover soft constraints may be incorporated. This incorporation may be by associating the atomic goals with default advice preferably held in the domain knowledge base. The default advice is advice which is specific to the user (that, is the individual who is the subject of the advice). For example, the default advice maybe input by the human advisor.

Once the atomic goals have been provided and optionally prioritised, they are processed by advice generation engines (for example in the form of software engines well known in the prior art) to generate advice. In invention embodiments, an engine finder is arranged to activate one or more selected advice generation engines. Preferably, the engine finder is arranged to read atomic goals, compare them to a plurality of advice generation engines, select suitable engines and produce engine activation instructions on the basis of the comparison to activate the suitable selected engines for use in advice generation specific to each atomic goal. If, however there is no selection involved by the engine finder, the preselected advice generation engines are fed with the atomic goals.

The system may further comprise an engine container component which reads the engine activation instructions and inputs atomic goals and knowledge from the domain knowledge base to invoke the suitable selected engines. This part is effectively an interface between the engine finder and the engines themselves: as mentioned above, the engines may be part of the system or external to the system.

The advice filter of invention embodiments is designed to check the advice before it is output to the user. Output may be by means of a print-out for example, or to a graphical user interface or via email or a web interface to a remote user. These interface elements can also be used for input by the health advisor/user.

The advice filter component may comprise an advice composition component and/or an advice compatibility checker component and/or a real time advice filter. Each of these individual parts may be arranged to operate separately and the advice may be fed to one only of them or fed to any combination of them in any order. The individual parts may be able to modify the advice. Thus there may be an advice generation cycle, in which advice filtered and perhaps modified by one individual part of the overall filter is fed back through one or more of the other parts, for re-checking. The cycle may end when no further changes have been made by any of the individual filter parts.

An advice composition component may be arranged to receive a list of advices from each suitable selected engine against each atomic goal, aggregate the advices from different lists and preferably to use critical factors stored in the domain knowledge base and/or domain knowledge stored in the domain knowledge base and/or the atomic goals to compose a ranked set of advices.

The advice composition component can realise the aggregated advice by adding more description to the aggregated advices. This may be achieved using domain knowledge from the domain knowledge base. Additionally or alternatively, the advice composition component can take into account the prioritised atomic goals which may be stored in storage separately from or within the domain knowledge base. The set of advices may be ranked using this input. Indeed, the engine may well already have weighted the advice with a weight or confidence level. Additional or alternative weighting may be computed from appropriate factors such as any combination of the importance of the atomic goals, the critical level of the associated risk factor, the confidence of the domain knowledge and the success rate of an action.

The system may use a subjective confidence level from experts and/or a confidence level from learning algorithms and/or a statistic confidence level from internet information. Advice composition may be carried out using a rank/linear aggregation algorithm to take into account numerical confidence levels and/or other weighting.

The advice compatibility checker component may be arranged to check advice compatibility across multiple atomic goals, to remove invalid advices and optionally to check if manual intervention is required. For example, two atomic goals may be in contradiction or they may be similar but include different parameters. The advice compatibility checker can choose the more appropriate advice in these circumstances.

The "real-time" advice filter is a component that validates advice against hard or mandatory constraints. The term "real-time" indicates that the filter can act as an updating methodology. In particular, current, data from one or more sensors can be incorporated into the system at this point, to be taken into consideration in checking and potentially modifying the advice. Also, input may be provided directly by the user, for example on his/her current physical and/or mental state. Potentially there may be a check against (up-to-date) mandatory advice from the human advisor for a particular individual. Data from third parties or public data may also be taken into consideration. If one or more of these constraints is not satisfied, it may be possible to modify the advice realisation, until the constraints are satisfied.

Finally, the advice filter component may include an advice personalisation component. This can be to take the user's personal preferences into account, either if they have been stored or through real time interaction with the user, for example, through a graphical user interface or connection to another system (such as by a web method or email). The graphical user interface or connection may also provide a negotiation so that there may be an interaction with the user (or individual) for justification, re-composition and re-ordering of advice.

According to an embodiment of a further aspect of the present invention there is provided a computer-implemented method in a healthcare system of producing personal advices, the method comprising receiving a healthcare goal and personal risk factors for a user and providing atomic goals from a combination of the personal risk factors and the healthcare goal; inputting the atomic goals into selected advice generation engines, which advice generation engines provide advices relating to the atomic goals; and filtering the advices from the selected reasoning engines against factors to provide checked advice for output.

According to an embodiment of a further aspect of the present invention there is provided a computer program which when downloaded onto a computer provides a healthcare system arranged to produce personal advices for a user, the system comprising a goal analyser arranged to receive a healthcare goal, to receive personal risk factors for a user and to provide atomic goals from a combination of the personal risk factors and the healthcare goal; an engine finder arranged to input atomic goals to selected advice generation engines, which provide advices relating to the atomic goals; and an advice filter, which filters the advices from the selected reasoning engines against checking factors to provide checked advice for output.

According to an embodiment of a further aspect of the present invention there is provided a computer program which when executed on a computer carries out the method of receiving a healthcare goal and personal risk factors for a user and providing atomic goals from a combination of the personal risk factors and the healthcare goal; inputting the atomic goals into selected advice generation engines, which advice generation engines provide advices relating to the atomic goals; and filtering the advices from the selected reasoning engines against factors to provide checked advice for output.

According to a yet further embodiment, there is provided a computer-implemented healthcare system arranged to produce personalised healthcare assistance for a user, the system comprising a goal analyser arranged to receive a healthcare goal, to receive personal risk factors for a user and to decompose the healthcare goal to provide atomic goals from a combination of the personal risk factors and the healthcare goal.

According to a yet further embodiment, there is provided a computer-implemented healthcare system arranged to produce personal advices for a user, wherein the system is arranged to receive a healthcare goal and personal risk factors for a user and to provide advices relating to the atomic goals; wherein the system comprises an advice filter which filters the advices against checking parameters to provide checked advices for output.

Features and sub-features of different aspects may be freely combined unless clearly incompatible. Thus the method and computer program aspects may be characterised by the sub-features of this system aspect as set out above.

In any of the above aspects, the various features may be implemented in hardware, or as software modules running on one or more processors. Features of one aspect may be applied to any of the other aspects.

The invention also provides a computer program or a computer program product for carrying out any of the methods described herein, and a computer readable medium having stored thereon a program for carrying out any of the methods described herein. A computer program embodying the invention may be stored on a computer-readable medium, or it could, for example, be in the form of a signal such as a downloadable data signal provided from an Internet website, or it could be in any other form.

Brief Description of the Drawings

Reference is made, by way of example only, to the accompanying drawings in which:

Figure 1     is a graph of obesity in the adult population;

Figure 2       is a diagrammatic illustration of risk factor data;

Figure 3       is a diagram of the components in a generalised embodiment;

Figure 4       is a flow chart of a generalised embodiment;

Figure 5       is a system diagram of a particular embodiment;

Figure 6       is a flow chart of a goal analyser;

Figure 7       is a flow chart of an engine finder;

Figure 8       is a flow chart of generation of a list of advices from reasoning engines;

Figure 9       is a flow chart of advice composition procedures;

Figure 10      is a flow chart of an advice compatibility checking stage;

Figure 11      is a flow chart of "real-time" advice filtering;

Figure 12      is a flow chart of a process for further personalising the advices; and

Figure 13      is an exemplary hardware implementation suitable for invention embodiments.

## General Description

One important factor in some invention embodiments is to be able to offer a range of "services" on an adaptable and scaleable platform that provides optimal value to stakeholders. This method can be applied for supporting operations and decision making about individual's lifestyle. The system would aid community services including intermediate care services or rehabs in assisting community nurses, health visitors, and individual patients to maintain their health & wellness.

Some of the most important risk factors for poor health in Europe include tobacco use, high blood pressure, obesity, high cholesterol, alcohol abuse, and low levels of physical activity, most of which are preventable by means of ensuring that healthy choices are available to individuals. Healthcare professionals such as community doctors or community care nurses can provide education and counselling in order to help people lead more active lifestyles. For this purpose, it is useful to establish personalised plans that are adapted to each individual as trust and relevance (personalisation) are among the many motivational factors. Activities that are tailored to individual interests and preferences can be more effective in increasing physical activity yet delivering promising results.

Such methods are needed that enable or support individuals in decision making as part of their daily lives. It is imperative to educate individuals about their health and it is important to obtain self-supported outcomes.

Despite the existence of several related-art personal planners and lifestyle recommendation systems, the personalisation aspect is overlooked; there is a need for a mechanism that incorporates real-time contextual data and/or individual preferences to generate dynamic and context-aware advices, for example whenever there is a change in an individual's situation or health status.

## Inventors' Identification of Problems or Inefficiencies

- Individuals cannot self-manage their health because they do not have better awareness on their lifestyles/lifestyle options or they lack the knowledge/expertise to associate lifestyle options with their targets. It would be good if the patients can self-manage their health and are have better awareness on their lifestyles/lifestyle options. There is a need to provide information that is of supreme relevance at a particular time (or at the time when it is needed). Such tools are needed that enable/support individual in decision making as part of their daily lives. It is imperative to educate patients about their health. It is important to obtain self-supported outcomes.
- Human advisor would like to provide complicated advices but have no time to do so as it is very labour intensive. If the advice does not take into consideration the individual's state, then they keep coming back for more advices. There is a need for such a service that would potentially help human advisors reach a wider population and reduce the number of revisits from the individuals.

- Individuals state and environment has huge impact on compliance, as with dynamic health state it becomes impossible to comply with fixed advices. Therefore, both (state and environment) must be considered while providing advices to individuals.
- Difficult to modify the advice taking into account the context data
- Difficult to generate an advice which requires different types of inference engines

From the above description, the inventors have derived the following issues:

1. A query to a knowledge base (or a goal one would like to achieve) can be too generic and too high level that cannot lead directly to concrete actions. Such goals need to be broken down into specific and single purposed sub goals simplifying the reasoning procedure and reducing the processing time needed for more complicated goals.
2. Data analytics draw conclusions from multiple information systems including sensor devices. Inferences involving the integration of potentially heterogeneous results may require a mechanism aligned with the original goals/queries. Risk factors should bear the signature of the original goals and guide the composition of reasoning results that can reduce the feedback loop to the original goals and reduce the number of interactions with the end users. Options (or actionable items) resulting from multiple inferences should preferably be dynamically updated using real-time data from sensors and devices.

Invention embodiments aim to address some of the above issues/problems and inefficiencies.

A method and system to generate real-time, dynamic, context-aware personalised advices can be based on an individual's situation in terms of health status, personal preferences, and context. It can improve the accuracy of advice available over prior art methodology. It can use personalised critical risk factors to decompose an individual's goal into atomic sub-goals and correlate dynamic advices to each of them. For each of the atomic goals, a suitable advice generation engine may be selected and the advices generated from each engine may be composed. The composed sets of advices can then be checked for compatibility against each atomic goal and (if needed) an approval sought from the human advisor. All valid advices can be filtered in real-time against mandatory constraints and external data (such as sensor data). Finally, advice personalisation can be performed to check against personal preferences and negotiating with the individual. The method and system can provide cost savings due to reduced re-visits of individuals to their care facilities for seeking advices when their situation changes, thus providing recommendations that allow individuals to make informed decision.

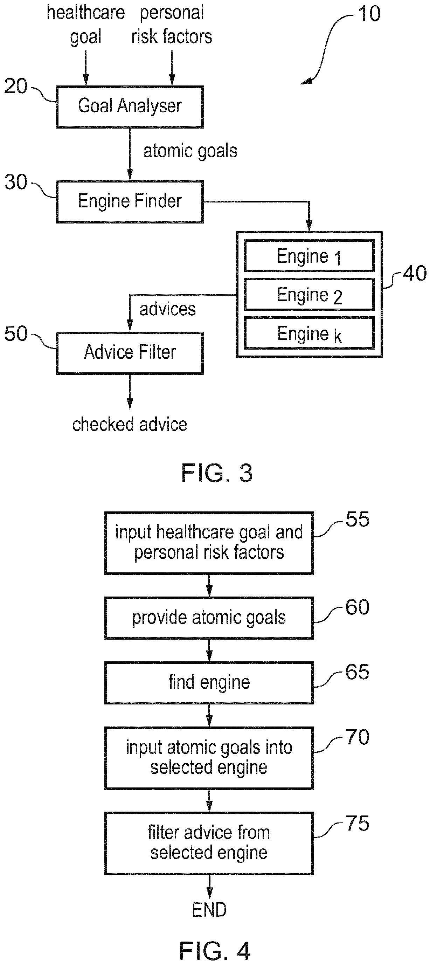

Figure 3 shows a functional representation of a system according to invention embodiments. The system comprises three main parts: goal analyser 20, engine finder 30 and advice filter 50. These processing parts may be provided within a common resource (such as a server or PC) or they may be spatially separated but interconnected. A healthcare goal and personal risk factors for a user are input into the goal analyser. The input may be automatic or manual, remote or within the healthcare system. In one preferred embodiment, a healthcare advisor or the individual inputs the healthcare goal but the personal risk factors are read from a domain knowledge base which may be held in computer storage either local or remote to the system. The goal analyser is essentially a processor which produces atomic goals. These atomic goals are passed to engine finder 30. The engine finder inputs the goals to selected advice generation engines 40. As mentioned before, the engines may be provided as part of the system, or external to the system. In either case they may be spatially local or remote. The selected engines provide advices relating to the atomic goals. These advices are then filtered by an advice filter 50. The advice filter may incorporate various checking factors to provide checked advice. For example the advice filter can aggregate and check advices from different engines, rank advices, check advice compatibility between different advices, validate the advices against various constraints and personalise the advices.

Figure 4 shows a simple flow chart of a method according to invention embodiments corresponding to the diagrammatic illustration of Figure 3. In step 55 the healthcare goal and personal risk factors are input. In step 60 atomic goals are provided. In step 65 one or more suitable engines are found and in step 70 atomic goals are input into the engine or engines. In step 75 the advice from the selected engine or engines is filtered.

## 1. Detailed Description

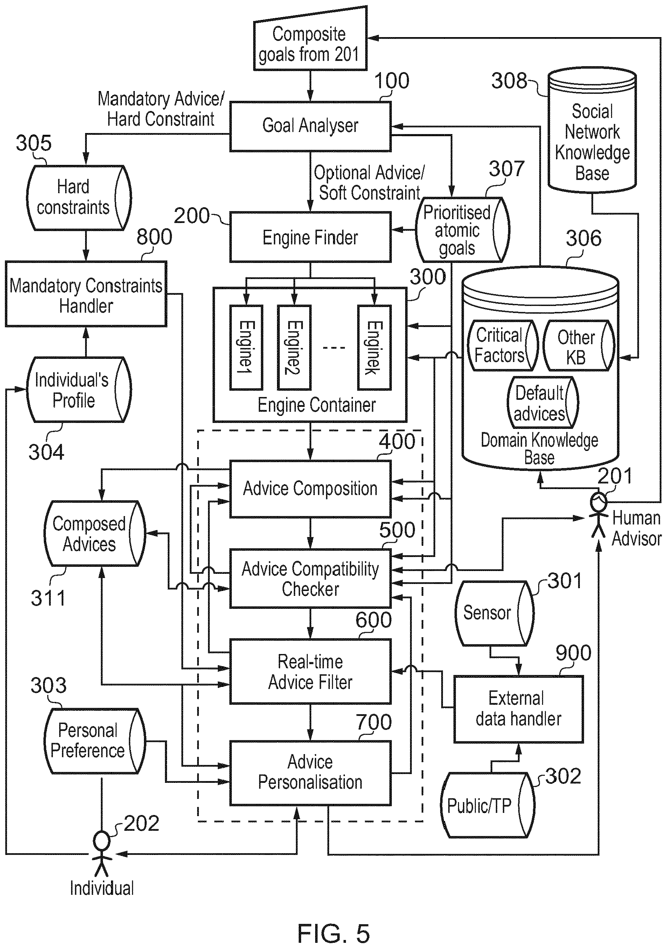

This section provides a detailed description of a method and system to generate real-time, dynamic, context-aware personalised advices based on the dynamic situation of an individual. This dynamic nature is due to the changes in health status, personal preferences, and/or the environmental contexts. Also, the changes in best practices or evidence-base contribute to the changing situation according to which personalised advices need to be modified. The system is depicted in Figure 5, which shows the interaction of various systems components.

Goal analyser 100 receives composite goals from human advisor 201 and personal risk factors for a user from the main knowledge base 306. It outputs prioritised atomic goals 307. The goal analyser may also output mandatory advice/hard constraints to hard constraints storage 205 as well as optional advice/soft constraints to engine finder 200. The engine finder 200 inputs the prioritised atomic goals along with optional advice/soft constraints then finds engines in engine container 300. The engine container inputs the prioritised atomic goals 307 along with information from domain knowledge base 306 to invoke the suitable selected engines. The engines output advices. These advices are then checked in the advice filter which is shown in a dashed rectangle. The advice filter comprises an advice composition element which receives the devices from the engine container and also receives input from other sources such as the domain knowledge base, the prioritised atomic goals and fed back advice from the advice compatibility checker 500 and real time advice filter 600. That is, the advices may be modified by these parts and then recomposed in advice composition. The advice composition element is linked to the advice compatibility checker, which receives inputs from the domain knowledge base, the prioritised atomic goals, input from a human advisor 201, input from the domain knowledge base 306, potentially the advisor 201 and of course the composed advices 311. Appropriate data is fed from advice composition 400 to advice compatibility checker 500, 500 to real-time advice filter 600 and 600 to advice personalisation 700. For example, multiple options pass from 400 to 500, multiple options excluding inappropriate or incompatible options pass from 50 to 600 and multiple options after filtering through hard and soft constraints pass from 600 to 700. The output from the advice compatibility checker is fed to composed advices 311 as well as to real-time advice filter 600 which has additional inputs from the mandatory constraint handler 800 using the hard constraints and storage 305, as well as an external data handler 900 which feeds in sensor data 301 and public/TP data (third party data) 302

Finally, the advice personalisation component receives input of composed advices from600 as well as from stored personal preferences 303, from the user 202 and from an individual's profile held in storage 304. The advice personalisation is shown outputting to the human advisor 201 And to the individual 202.

The method of invention embodiments may comprise the following operations:

**Operation 1: Analyze the overall goals and decompose them into sub-goals using personalised risk factors**

Risk factors are utilised as the guidance of goal decomposition. Normally, a goal given to the system is relatively broad and high level, preventing a straightforward answer or comprising multiple sub goals. In order to situate a generic goal into individual's situation, goals need to be decomposed into atomic ones. The decomposition is done using personal risk factors.

Goal decomposition relies heavily on domain knowledge. It is assumed that following types of domain knowledge are available:

• *knowledge of what actions can be taken with respect to a goal:*

For instance, the goal [hypertension management] implies that one can do one or all of the followings:

    a. Maintain a good sleep pattern
    b. Reduce daily sodium consumption
    c. Increase daily water consumption
    d. Reduce alcohol consumption
    e. Reduce cigarette consumption
    f. Etc.

All of these are universal and can be applied to anyone having "hypertension management"

Most of the knowledge is readily available from WWW and/or from healthy living leaflets easily accessible from family doctors/general practitioners. Modelling and representation of such knowledge is provided in the system. Information may be stored in a suitable form in the domain knowledge base, which is a repository of relevant healthcare information from various sources. Some part of the domain knowledge base may be created from hospital systems (such as EMRs - Electronic Medical Records) and the human advisor. *Knowledge of personal risk factors:*

Such information can be acquired from human health advisors. Invention embodiments do not aim to automatically infer personal risk factors without intervention by human experts. An individual's risk factors can be obtained from the most recent consultation with his/her health advisors. In invention embodiments, a risk factor may be dynamically adjusted. Individual's status is constantly monitored by sensors (e.g. weight, heart rate sensors) and by dietary and activity diaries. Should signs of changing risk factors can be detected from such data, human health advisors are informed, who subsequently adjust the defined risk factors according to the data. Highlighting and informing individuals with risk factors is widely practiced for example by General Practice in the UK and individual's situation is periodically reviewed through health screening.

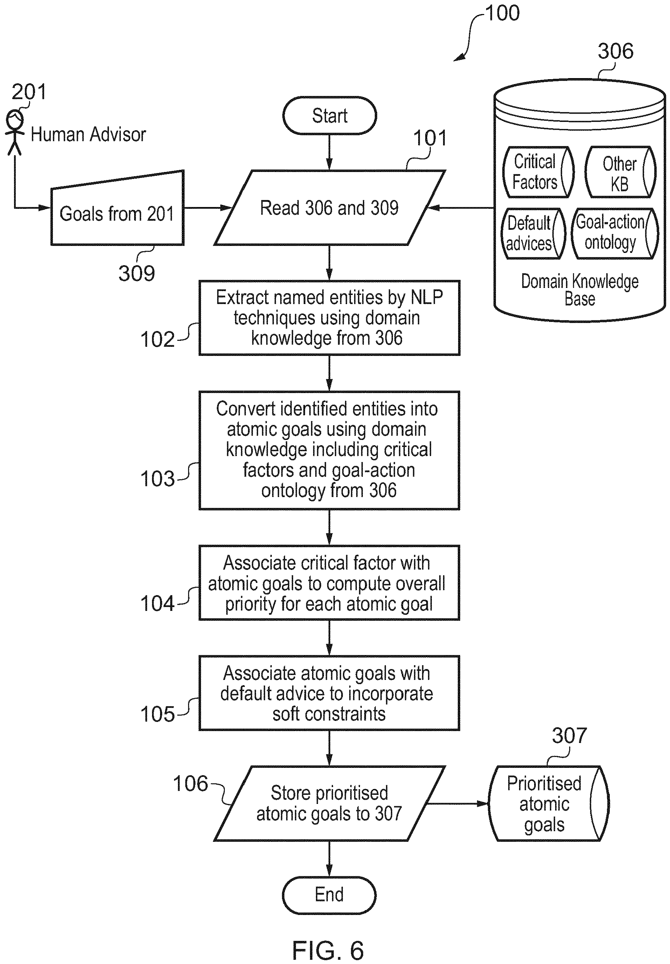

Figure 6 shows an example of how critical/risk factors, together with other knowledge bases (KB) and a "goal-action ontology", can help to decompose and prioritise a composite goal into more concrete and specific atomic goals. A goal-action ontology is a structured relationship between a goal and actions that can contribute to the goal. It is pre-created (before the system is running) by public domain knowledge and input from the human advisor. The goal-action ontology may be updated while the system is in operation.

Decomposition is performed based on the following algorithm.

```
function Decomposition (G, O, R) {
    G, O → {<action, significance>};
    for each action pair A in {<action, significance>}
        for each C in R
            if A matches C
                compute the accumulated overall weight of A, WA(G,R)
            end if
        end for
    end for
end function
```

The decomposition algorithm takes as input the goal (G) to be achieved, the goal-action ontology (O) and a set of personal risk factors (R). It first retrieves all the actions that can contribute to the given goal with a numeric number quantifying the level of contribution. Such numeric value can come from different sources. For instance, it can be given by domain experts when defining the ontology, can be acquired from the literature (e.g. pubMed), can be computed based on statistic methods using past experience, etc. When retrieving the actions, ontological inheritance is observed along the subsumption hierarchies.

Contributing action (C) is the causal action towards a risk factor, i.e. an action that would help decrease that risk factor. It corresponds to the beneficial activity shown in Figure 2. The contributing actions of goals and the personal risk factors are then compared to identify overlapping ones. Overlapping is not simply string-matching, even though a simple string-matching algorithm can become helpful. An action can contribute to the management of multiple risk factors and vice versa.

Once overlapping is found, the significance level of actions, that is, their causal effect, and the critical level of risk factors are then aggregated to produce the final weights of actions. Atomic goals are created for actions having weight value over a threshold (given by domain experts). This threshold can be input by a human advisor. These are sub-goals derived from the original goal G for the target individual.

When aggregating lists, standard methods, e.g. standard averaging or sigmoid function when multiple actions are associated with a risk factor (with certain degree of fuzziness), can be used.

The flowchart of goal analyser and decomposition (100) is depicted in Figure 6.

- 101: The goal analyser component 100 reads goals from 309 and personalised risk factors from the domain knowledge base 306 in order to generate atomic and operable sub-goals:

    - 306 contains the domain knowledge including critical risk factors and default advices provided by domain experts and knowledge from the social network knowledgebase
    - 309 is the manual input from human advisor (nurse, doctor, pharmacist, etc.) entering goals for a particular individual or input from individuals in cases of self-management

- 102 & 103: The goal analyser component 100 then extracts named entities from the goal and maps them to the knowledge base, using known NLP techniques to locate relevant portions of text that might be atomic goals by using Text Analysis techniques and converts identified entities into atomic goals using the domain knowledge from 306.
- 104: The goal analyser component 100 can associate universal risk/critical factors (example shown in Table 1 below) with the atomic goals to compute the overall priority for each atomic goal

- 105: The goal analyser component 100 can incorporate soft constraints by associating the atomic goals with default advices from 309. Soft constraints may comprise rules set by the human advisor (e.g. if blood pressure>140 then monitor every two hours for the next 12 hours). The previous operations provide atomic goals which may be prioritised.
- 106: The goal analyser component 100 stores the (risk factor based prioritised) atomic goals to 307 in order to perform goal-driven advice composition

  - 307 contains prioritised atomic goals that are measurable and are used throughout the system

**Table: 1 Example of some of the most important critical (risk) factors for poor health in EU**

| Critical Factors |
| --- |
| Tobacco |
| Blood Pressure |
| Alcohol |
| Overweight |
| Cholesterol |
| Physical inactivity |
| Low fruit & vegetable intake |
| Illicit drugs |

**Operation 2: Select engine for advice generation based on prioritised atomic goals**

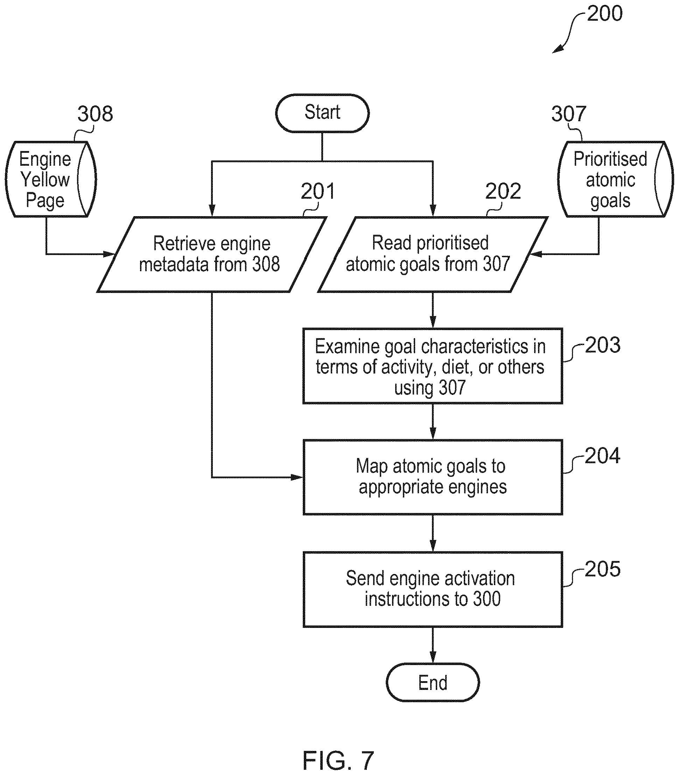

A flowchart for engine selection and activation is depicted in Figure 7.

- 201 & 202: The engine finder component 200 reads prioritised atomic goals from 307 and retrieves engine metadata from 308:

  - 308 is a directory of engine information relating to available engines ("Engine Yellow Pages"); it contains metadata about each of the engines contained within the engine container component 300. Such engines commercially available. They may be external to the system, or internal.

- 203: The engine finder component 200 then examines the characteristics of each atomic goal in terms of activity, diet, or others from 307
- 204: The engine finder component maps atomic goals to appropriate engines
- 205: The engine finder component 200 sends engine activation instructions for each engine to the component 300. Thus the engine finder component 200 delivers atomic goals to the appropriate engine.

**Operation 3: Generate ordered list of advices from each engine against each atomic goal**

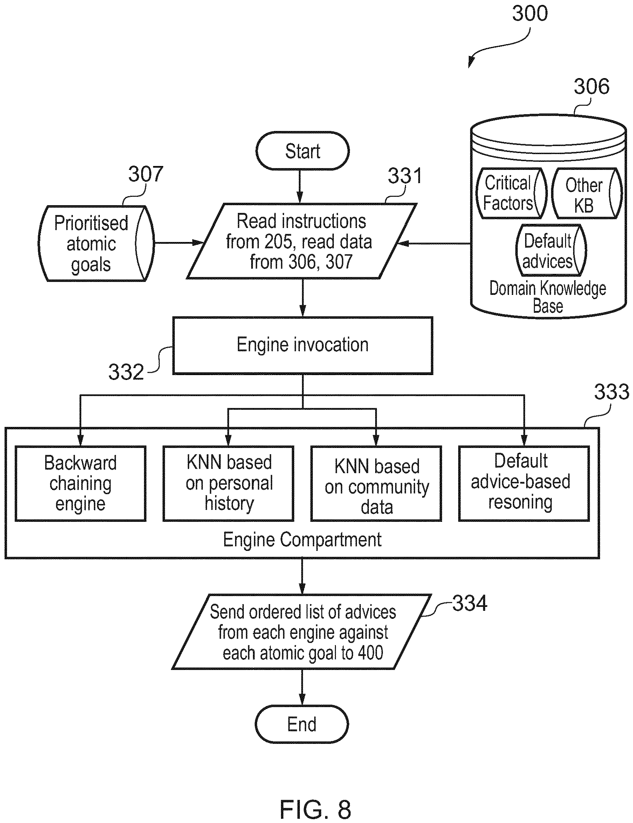

A flowchart for the generation of this list is depicted in Figure 8.

- 331: The engine container component 300 is a class. It reads engine activation instructions from 205. It also reads the domain knowledge base 306 and prioritised atomic goals from 307
- 332: The engine invocation component 332 then invokes the advise generation engines within the engine compartment 333 in accordance to the respective atomic goals
- 334: An ordered list of advices from each of the engines against each atomic goal is sent to the component 400 by component 334. The atomic goals may be delivered to more than one engine.

**Operation 4: Compose advices generated from multiple engines**

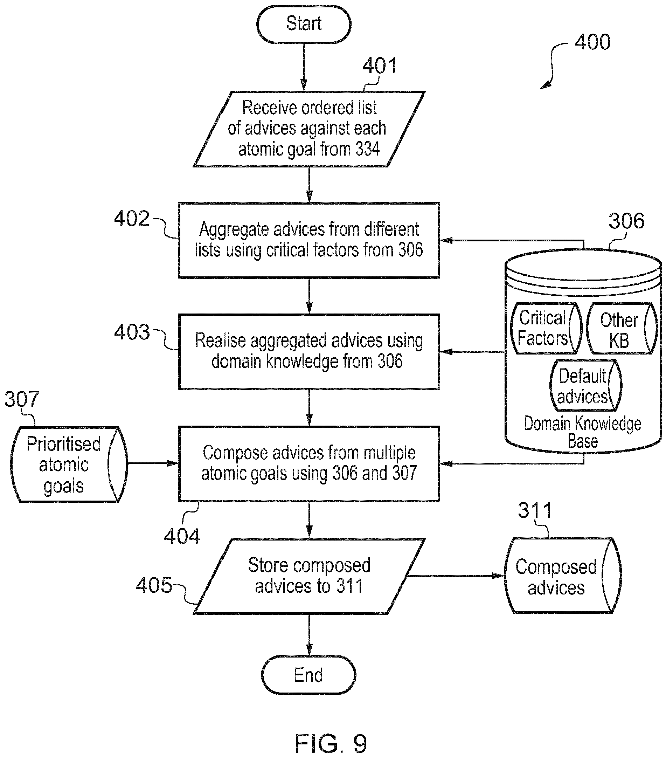

A flowchart for advice composition is depicted in Figure 9

- 401 & 402: The advice composition component 400 receives the ordered list of advices against each atomic goal from 334 in process 401; it then aggregates advices from different lists using risk factors from 306

- In process 403, the advice composition component 400 realises the aggregated advices by using domain knowledge from 306 to incorporate further information.
- 404: The advice composition component 400 composes advices from multiple atomic goals using 306 and 307
- 405: The advice composition component 400 stores composed advices to 311 for validation

  - 311 contains composed advices that are used throughout the advice generation cycle loops, including the loop described above for Figure 5.

Personal risk factors also play an important role when composing advices returned from different engines, or in cases of multiple goals, computing integrated advices across different goals. For instance, a "tuning body" goal implies multiple sub-(atomic)-goals, each independently leading to a set of advices that might overlapping, even contradicting, with the advices derived from other atomic goals. The system aims to present an intelligently composed version (well-informed inferences) to give users easy-to-follow, straightforward advices.

Advices given by the engines are normally associated with a weight or a confidence level when learning algorithms are used. Weights of advices, if not computed directly from the learning algorithms, come from the following sources:

- N1: Importance of the atomic goals (computed when decomposing a goal)
- N2: Critical level of the associated risk factors (pre-defined against each individual)
- N3: Confidence level of the domain knowledge (given by the domain experts)
- N4: Success rate of an action (learnt from WWW)

It is worth noting that the system exercises a subjective confidence level when soliciting the domain knowledge from experts or a statistic confidence level when pulling domain data from the WWW. In both case, the level is represented as a numeric value and treated as the baseline confidence of the advice, which is further adjusted according to N1 and N2.

Advice composition can be boiled down to a rank aggregation problem.

Apart from the standard rank aggregation algorithms, advices with multiple confidence values are processed as followings: where raw advices are represented as a tuple $<adv, <ag, rf>, w>$, $ag$ stands for atomic goal which the advice aims to address, $rf$ the risk factor, and $w$ the weight as described in the previous section.

We define:

$AG = \{ <ag, rf> \square\ G\}$ as all the risk factor bound atomic goals of $G$.
$Adv = \{ <ag, rf> \rightarrow e\ advice\ |\ <ag, rf> \square\ AG\}$
$W_{adv} = F<ag, rf> \square\ AG\ (<adv, <ag, rf>, w>)$.

Again, linear aggregation (possibly adjusted with sigmoid function (as shown below)) can be used to instantiate function F(). When $w$ can be converted or normalised to proper probabilities, standard information entropy methods could also be applied.

$$P(t) = \frac{1}{1 + e^{-t}}.$$

The sigmoid function is widely used to integrate multiple sources of evidence. This is the multiple sources of advice generation engines supporting or against a particular advice. The rationale behind using sigmoid adjustment is to emphasise on advices with high confidence values and decrease the impact of those with very low confidence. In order to shift the function curve into the range of 0...1, the standard sigmoid function should be adjusted by -0.5, i.e. F (t) = P(t-0.5).

This processing is a new and advantageous feature of invention embodiments.

**Operation 5: Check advice compatibility against each atomic goal and seek approval from the human advisor**

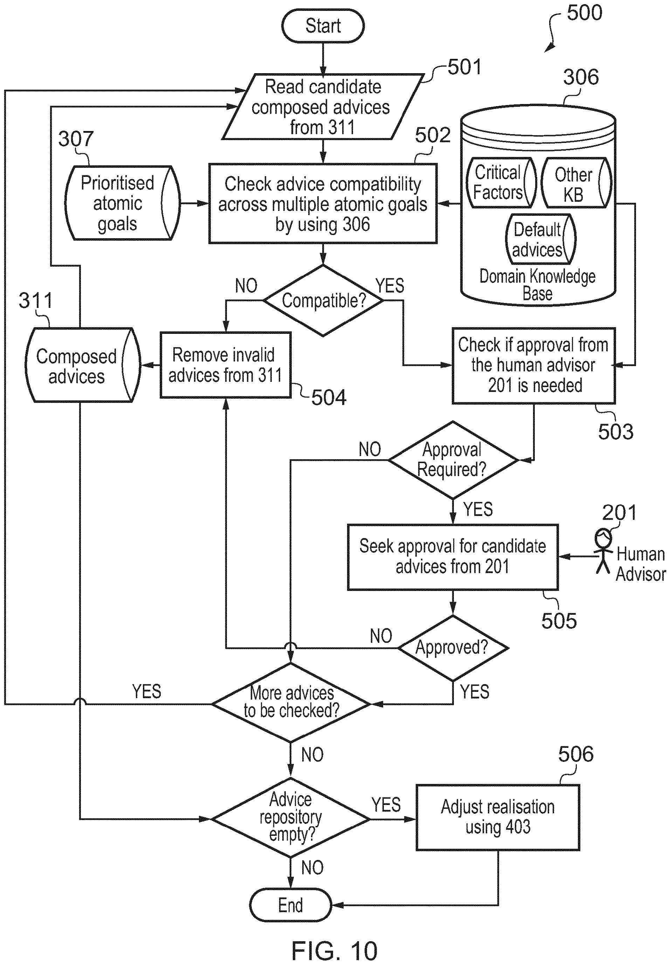

A flowchart for checking advice compatibility is depicted in Figure 10.

- 501: The advice compatibility checker component 500 reads the candidate composed advices from 311
- 502: The advice compatibility checker component 500 checks advice compatibility across multiple atomic goals by using data from 307 and 306

- If the advices are compatible, then 503 would check if an approval from the human advisor (201) is needed depending on the nature of advice
- If the advices are not compatible or are not approved by the human advisor, then 504 would remove the invalid advice from 311 and read the next advices for compatibility check

• The advice compatibility checker component 500 checks for more available advices until all advices are checked and only valid advices remain in 311

Advices generated by multiple engines might present certain extent of disagreement. The incompatibility is resolved as followings:

1. The numeric values associated with individual advices are processed and the maximum value is treated as the final realisation of compatible advices. For example, when both "running 20 minutes" and "running 10 minutes" exist, "20 minutes" will be used to realise the activity "running".
2. When activities are different but not conflict with each other, the activity with the maximum effect will be selected. Domain knowledge will be consulted to evaluate the impact of activities. For example, if both "mall walking 30 minutes" and "jogging 20 minutes" exist, assuming that the system understands from the domain knowledge that jogging will contribute more towards achieving the goal, "jogging 20 minutes" will be selected. Such domain knowledge should be elicited and readily captured in 306.
3. When activities conflict with each other, they are filtered based on the weights associated with them. The higher ranking activities will automatically deny lower ranking and contradictory activities.
4. It can be critical that human advisors are consulted when the above changes are made, to ensure "no harm" will be done to the advice receiving party (individual user of the system).

**Operation 6: Perform real-time filtering of the composed advices against various parameters, such as hard constraints and external data**

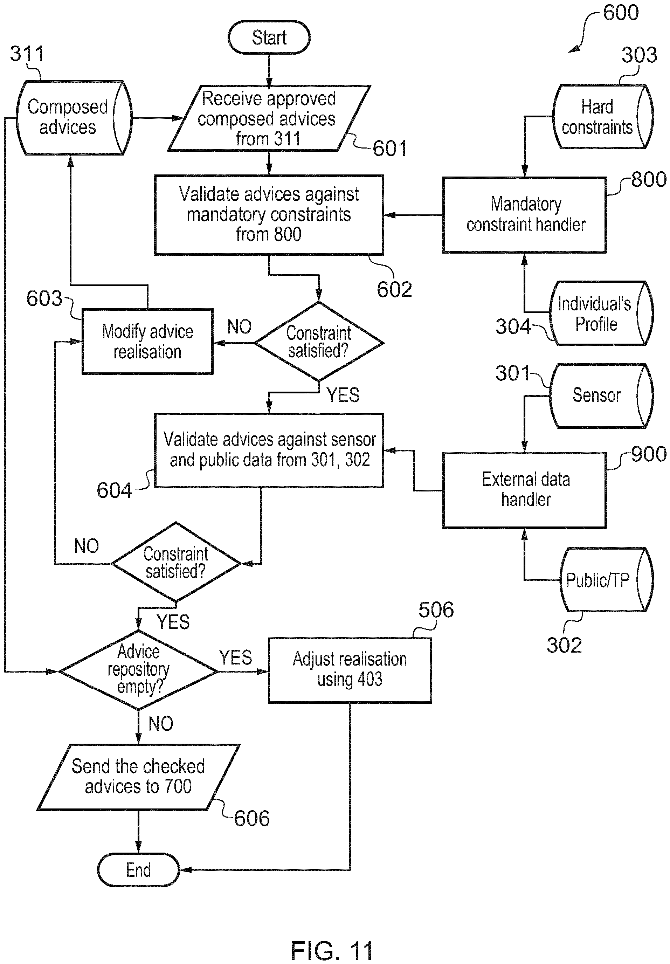

A flowchart for filtering is depicted in Figure 11.

• 601: The real-time advice filter component 600 reads the approved composed advices from 311
• 602: The real-time advice filter component 600 then validates the composed advices from 311 against hard or mandatory constraints from 305

- 305 is the mandatory constraint handler that manages data form 303 and 304
- 303 contains the hard constrains or mandatory advices from the human advisor (201) for a particular individual
- 304 contains individual's profile for the personalisation of advices.

• 604: The real-time advice filter component 600 then validates advices against the sensor or public data from external data handler 900

- 900 is the external data handler that manages data form 301 and 302
- 301 contains data from various sensors including physiological
- 302 contains data from public or third party such as environmental sensors data

• The real-time advice filter component 600 checks if both the constrains are satisfied:

- If constrains are satisfied, then the approved or valid advice would be sent to 700
- If constrains is not satisfied, then 504 would read the next advices for compatibility check against real-time data from 301 and 302

In this operation, generated and checked advices may be further processed by considering other types of parameters, for example hard constraints and real-time individual data.
Hard constraints are those that presenting the known and long-term physical / mental constraints of the subject individual. These might be given by the human advisor knowing the individual's status or documented historical data of the individual including the risk factors. Examples include "no more than 2 cigarettes per day" or "knee damage". Such constraints are used to filter the advices. For instance, "knee damage" will exclude all the physical activities that may involve knees. Domain knowledge from 306 is used to establish necessary correlations. We utilise a human model ontology and activity impact ontology to connect human body parts with activities. For instance, *running* as an activity involves excessive *lower limb movement,* significant *vertical impact on human body,* moderate *upper limb/shoulder movement,* etc., while *knee* is **part-of** *lower limb.* The system is then able to conclude that *running* and *knee damage* should be exclusive to avoid any harm to the individual.

Real-time individual data come from two different sources. The users can push information in to the system by using an interactive interface to inform the system his/her feeling at the point of using the system. On the other hand, the system is able to pull information from surrounding sensors to collect real time environmental and bio data. For example, environmental sensors might detect a very high outdoor temperature and very high UV index and thus reduce the weights of all the outdoor physical activities by a predefined factor a. This is to avoid any potential damage to the individual, should he/she be exposed to unnecessary risks leading to hyperthermia or skin damage. As a result, such activities will be moved towards the bottom of the list.

**Operation 7: Personalise advices and validate against personal preferences**

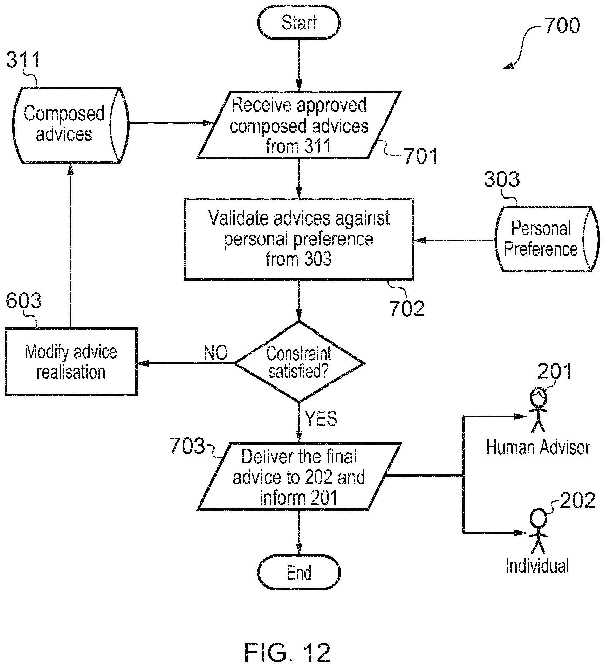

A flowchart for personalisation and validation is depicted in Figure 12.

- 701: The advice personalisation component 700 reads the approved composed advices from 600
- 702: The advice personalisation component 700 adjusts advice realisation against the personal preferences from 303 or real-time interaction with 202 through a graphic user interface

  - 303 contains an individual's personal preferences or lifestyle choices; this provides flexibility, personalisation, and empowerment to the individual

    + 703: The advice personalisation component 700 then delivers the final validated advices to the individual (202)

      o If individual does not accept the advices, then a negotiation is performed in terms of interaction with individual for justification, re-composition, and reordering of advices until an agreement is reached
      o If individual accepts the advices, then the human advisor is informed about the new advices

**Overview of Hardware Implementation**

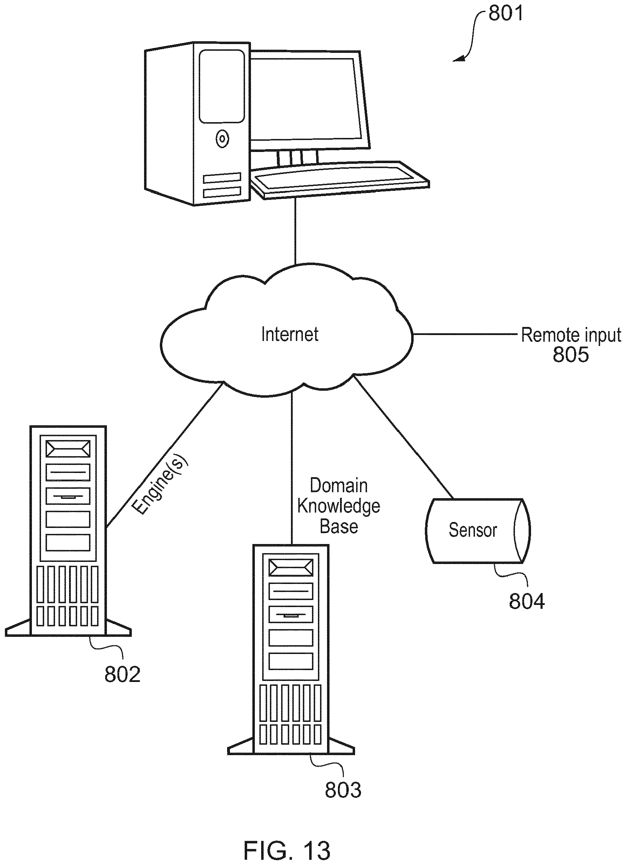

Figure 13 gives an overview of one exemplary hardware implementation, although many different hardware implementation would be feasible. Figure 13 shows the system of invention embodiments 801 including a server, with graphical user interface. A server can provide the goal analyser, engine finder and advice filter previously described, as well as the reasoning engines previously described. The system 801 comprises storage which can store data, such as the domain knowledge base, prioritised atomic goals, social network knowledge base, hard constraints and individual profile, composer advices, personal preferences and other data. Alternatively, any or all of this storage may be remote, such as provided over the internet. Figure 13 shows a remote domain knowledge base 803. It also shows a remote sensor 804 and the possibility of remote input by the user and/or healthcare advisor 805. One or more reasoning engines 802 may also be provided remotely.

**Some Distinctions over the prior art**

The technical effects of embodiments of the above described invention include efficient processing of knowledge base data giving:

- Provision of a Risk-factor based goal analysis, including

  o Goal-driven advice composition

- Provision of real-time advice filtering by using static and dynamic data
- Interaction with individual for justification, re-composition, and reordering of advices
- Complex advice composition by considering social network knowledgebase

**Benefits and Usage**

1. Embodiments of the method and system enable human advisor to provide more effective (and complicated) advices to the individual.

2. Embodiments of the method and system generate simple and hassle-free personalised advices considering the context and reflecting the current status of the individual.

3. Embodiments of the method and system can be applied to proactive wellbeing and self-management.

4. Optional constraints in invention embodiments allow more flexibility.

5. Embodiments of the invention reduces cost due to user (patient) revisits.

6. The engine according to invention embodiments can perform analytics for an individual in order to generate advices enabling inspired and informed 'self trackers'.

7. A user-centric interactive system can be produces for negotiation-based lifestyle or wellbeing guidance with multiple inferences.

8. The method and system according to invention embodiments would aid community services including intermediate care services or rehabs in assisting community nurses, health visitors, and individual patients to maintain their health & weliness.

9. The method and system according to invention embodiments provides information to help people live healthier lives; it provides information that is of supreme relevance at a particular time (or at the time when it is needed) thus supporting individual in decision making as part of their daily lives and obtaining self-supported outcomes.

10. The method and system according to invention embodiments support operations and decision making for the individual, e.g. self-management and Long Term Conditions (LTCs) management by providing integrated situational awareness, such as, combining lifestyle information with clinical data to provide risk assessment of lifestyle choices, prevention and early detection of disease, exercise regime and diet management, or access to peer communities.

## Claims

1. A computer-implemented healthcare system (10) arranged to produce personal advices for a user, the system comprising:

   a goal analyser (20) arranged to receive a healthcare goal, to receive personal risk factors for a user and to provide atomic goals from a combination of the personal risk factors and the healthcare goal;
   an engine finder (30) arranged to input atomic goals to one or more selected advice generation engines (40), which provide advices relating to the atomic goals; and
   an advice filter (50), which filters the advices from the selected reasoning engines against checking parameters to provide checked advice for output.

2. A system according to claim 1, wherein the goal analyser (20) is arranged to analyse the healthcare goal and the personal risk factors in conjunction with a goal-action ontology, and produce a list of actions, which is preferably weighted according to the level of contribution of the action on the personal risk factors and the significance of the personal risk factors for the user; and preferably to select actions having a weight value above a threshold as atomic goals.

3. A system according to claim 2, wherein the goal analyser (20) is arranged to use text analysis to extract actions from a domain knowledge base.

4. A system according to claim 2 or 3, wherein the goal analyser further prioritises the atomic goals and optionally incorporates soft constraints to provide prioritised atomic goals.

5. A system according to any of the preceding claims, wherein the engine finder (30) is arranged to read atomic goals, compare them to a plurality of advice generation engines, select suitable engines and produce engine activation instructions on the basis of the comparison to activate the suitable selected engines for use in advice generation specific to each atomic goal.

6. A system according to claim 5, wherein the system further comprises an engine container component (300) which reads the engine activation instructions and inputs atomic goals and knowledge from the domain knowledge base to invoke the suitable selected engines.

7. A system according to any of the preceding claims, wherein the advice filter includes an advice composition component (400) arranged to receive a list of advices from each suitable selected engine against each atomic goal, aggregate the advices from different lists and preferably to use critical factors stored in the domain knowledge base and/or domain knowledge stored in the domain knowledge base and/or the atomic goals to compose a ranked set of advices.

8. A system according to claim 7 wherein the rank of each advice is dependent on the importance of the associated atomic goal, the significance of the personal risk factor, and optionally also a confidence level of domain knowledge and/or a learned success rate of the action.

9. A system according to any of the preceding claims, wherein the advice filter component includes an advice compatibility checker component (500) arranged to check advice compatibility across multiple atomic goals, to remove invalid advices and optionally to check if manual intervention is required.

10. A system according to any of the preceding claims, wherein the advice filter component includes a real-time advice filter (600) arranged to validate the advices against constraints and/or current sensor data and/or public data; and optionally arranged to modify advice realisation as required by the validation.

11. A system according to any of the preceding claims, wherein the advice filter component includes an advice personalisation component (700) arranged to compare and refine advices against personal preferences of the user, and optionally also arranged to initiate at least one new advice generation cycle to take the personal preferences into account.

12. A computer-implemented method in a healthcare system of producing personal advices, the method comprising:

receiving a healthcare goal and personal risk factors for a user and providing atomic goals from a combination of the personal risk factors and the healthcare goal;
inputting the atomic goals into selected advice generation engines, which advice generation engines provide advices relating to the atomic goals; and
filtering the advices from the selected reasoning engines against parameters to provide checked advice for output.

13. A computer program which when downloaded onto a computer provides a healthcare system arranged to produce personal advices for a user, the system comprising:

a goal analyser arranged to receive a healthcare goal, to receive personal risk factors for a user and to provide atomic goals from a combination of the personal risk factors and the healthcare goal;
an engine finder arranged to input atomic goals to selected advice generation engines, which provide advices relating to the atomic goals; and
an advice filter, which filters the advices from the selected reasoning engines against checking parameters to provide checked advice for output.

14. A computer program which when executed on a computer carries out the method of:

receiving a healthcare goal and personal risk factors for a user and providing atomic goals from a combination of the personal risk factors and the healthcare goal; inputting the atomic goals into selected advice generation engines, which advice generation engines provide advices relating to the atomic goals; and
filtering the advices from the selected reasoning engines against parameters to provide checked advice for output.

15. A computer-implemented healthcare system (10) arranged to produce personalised healthcare assistance for a user, the system comprising:

a goal analyser (20) arranged to receive a healthcare goal, to receive personal risk factors for a user and to decompose the healthcare goal to provide atomic goals from a combination of the personal risk factors and the healthcare goal.

16. A computer-implemented healthcare system (10) arranged to produce personal advices for a user, wherein the system is arranged to receive a healthcare goal and personal risk factors for a user and to provide advices relating to the atomic goals;

wherein the system comprises
an advice filter (50), which filters the advices against checking parameters to provide checked advices for output.

**Amended claims in accordance with Rule 137(2) EPC.**

**1.** A computer-implemented healthcare system (10) arranged to produce personal advices for a user, the system comprising:

a goal analyser (20) arranged to receive a healthcare goal related to improvement or maintenance of a medical condition or illness, to receive personal risk factors for a user and to provide atomic goals from a combination of the personal risk factors and the healthcare goal;
an engine finder (30) arranged to input atomic goals to one or more selected advice generation engines (40), which provide advices relating to the atomic goals; and
an advice filter (50), which filters the advices from the selected advice generation engines (40) against checking parameters including advice compatibility with other advices to modify the advice and provide checked advice for output, wherein
the goal analyser (20) is arranged to analyse the healthcare goal and the personal risk factors in conjunction with a goal-action ontology, and produce a list of actions, which is weighted according to the level of contribution of the action on the personal risk factors and the significance of the personal risk factors for the user; and to select actions having a weight value above a threshold as atomic goals, and wherein
the engine finder is arranged to select suitable advice generation engines (40) on the basis of the atomic goals.

**2.** A system according to claim 1, wherein the goal analyser (20) is arranged to use text analysis to extract actions from a domain knowledge base.

**3.** A system according to claim 1 or 2, wherein the goal analyser further prioritises the atomic goals and optionally incorporates soft constraints to provide prioritised atomic goals.

**4.** A system according to any of the preceding claims, wherein the engine finder (30) is arranged to read atomic goals, compare them to a plurality of advice generation engines, select suitable engines and produce engine activation instructions on the basis of the comparison to activate the suitable selected engines for use in advice generation specific to each atomic goal.

**5.** A system according to claim 4, wherein the system further comprises an engine container component (300) which reads the engine activation instructions and inputs atomic goals and knowledge from the domain knowledge base to invoke the suitable selected engines.

**6.** A system according to any of the preceding claims, wherein the advice filter includes an advice composition component (400) arranged to receive a list of advices from each suitable selected engine against each atomic goal, aggregate the advices from different lists and preferably to use critical factors stored in the domain knowledge base and/or domain knowledge stored in the domain knowledge base and/or the atomic goals to compose a ranked set of advices.

**7.** A system according to claim 6 wherein the rank of each advice is dependent on the importance of the associated atomic goal, the significance of the personal risk factor, and optionally also a confidence level of domain knowledge and/or a learned success rate of the action.

**8.** A system according to any of the preceding claims, wherein the advice filter component includes an advice compatibility checker component (500) arranged to check advice compatibility across multiple atomic goals, to remove invalid advices and optionally to check if manual intervention is required.

**9.** A system according to any of the preceding claims, wherein the advice filter component includes a real-time advice filter (600) arranged to validate the advices against constraints and/or current sensor data and/or public data; and optionally arranged to modify advice realisation as required by the validation.

**10.** A system according to any of the preceding claims, wherein the advice filter component includes an advice personalisation component (700) arranged to compare and refine advices against personal preferences of the user, and optionally also arranged to initiate at least one new advice generation cycle to take the personal preferences into account.

**11.** A computer-implemented method in a healthcare system of producing personal advices, the method comprising:

receiving a healthcare goal related to improvement or maintenance of a medical condition or illness and personal risk factors for a user and providing atomic goals from a combination of the personal risk factors and the healthcare goal;

inputting the atomic goals into selected advice generation engines, which advice generation engines provide advices relating to the atomic goals; and

filtering the advices from the selected advice generation engines against parameters including advice compatibility with other advices to modify the advice and provide checked advice for output, wherein

the goal analyser (20) analyses the healthcare goal and the personal risk factors in conjunction with a goal-action ontology, and produces a list of actions, which is weighted according to the level of contribution of the action on the personal risk factors and the significance of the personal risk factors for the user; and selects actions having a weight value above a threshold as atomic goals, and wherein

the engine finder selects suitable advice generation engines (40) on the basis of the atomic goals.

**12.** A computer program which when executed on a computer carries out the method of claim 11.

United States(*) (1978-2008)
33.8% (+125.3%)
15.0%

United Kingdom(*) (1980-2008)
24.5% (+250.0%)
7.0%

Sweden (1989-2007)
10.2% (+85.5%)
5.5%

Spain (1987-2009)
17.1% (+151.5%)
6.8%

Netherlands (1981-2009)
11.8% (+131.4%)
5.1%

Japan (*) (1978-2008)
3.4% (+61.9%)
2.1%

Italy (1994-2008)
9.9% (+41.4%)
7.0%

Germany (1999-2009)
16.0% (+39.1%)
11.5%

France (1990-2008)
11.2% (+93.1%)
5.8%

Finland (1978-2008)
15.7% (+137.9%)
6.6%

Austria (1991-2006)
12.4% (+45.9%)
8.5%

latest available year
baseline year

0.0%  5.0%  10.0%  15.0%  20.0%  25.0%  30.0%  35.0%  40.0%

## FIG. 1

Personal risk factors:

Condition-Risk Factor Ontology data

| Condition | Weight for the individual | Risk Factor |
|---|---|---|
| Hypertension | 0.1 | Salty food |
| | 1.0 | Smoking |

Goal-Action Ontology data

| Composite goal | Level | Contributing Action |
|---|---|---|
| Hypertension Management | 0.3 | Eat non-salty food |
| | 1.0 | Quit smoking |

Prioritized atomic goals for the individual

| Composite goal | Level and Weight | Atomic goal |
|---|---|---|
| Hypertension Management | 1.0 | Quit smoking |
| | 0.2 | Eat non-salty food |

## FIG. 2

healthcare    personal
goal          risk factors

20 — Goal Analyser

atomic goals

30 — Engine Finder

40 —
- Engine 1
- Engine 2
- Engine k

advices

50 — Advice Filter

checked advice

10

FIG. 3

55 — input healthcare goal and personal risk factors

60 — provide atomic goals

65 — find engine

70 — input atomic goals into selected engine

75 — filter advice from selected engine

END

FIG. 4

FIG. 5

FIG. 6

200

308

Engine
Yellow
Page

Start

307

Prioritised
atomic
goals

201

Retrieve engine
metadata from 308

202

Read prioritised
atomic goals from 307

Examine goal characteristics in
terms of activity, diet, or others
using 307
203

Map atomic goals to
appropriate engines
204

Send engine activation
instructions to 300
205

End

FIG. 7

300

306

Start

307

331

Prioritised
atomic
goals

Read instructions
from 205, read data
from 306, 307

Critical
Factors

Other
KB

Default
advices

Domain Knowledge
Base

Engine invocation

332

333

Backward
chaining
engine

KNN based
on personal
history

KNN based
on community
data

Default
advice-based
resoning

Engine Compartment

Send ordered list of advices
from each engine against
each atomic goal to 400

334

End

FIG. 8

FIG. 9

FIG. 10

FIG. 11

311

Composed
advices

Start

700

Receive approved
composed advices
from 311
701

Validate advices against
personal preference
from 303
702

303

Personal
Preference

603

Modify advice
realisation

NO

Constraint
satisfied?

YES

201

Human Advisor

703

Deliver the final
advice to 202 and
inform 201

202

Individual

End

FIG. 12

801

Internet

Remote input
805

Engine(s)

Domain
Knowledge
Base

Sensor

804

802

803

FIG. 13

Europäisches
Patentamt

European
Patent Office

Office européen
des brevets

**EUROPEAN SEARCH REPORT**

Application Number

EP 11 19 1630

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/125680 A1 (BOSWORTH ADAM [US] ET AL) 26 May 2011 (2011-05-26)<br>* abstract; figure 1 *<br>* paragraph [0025] - paragraph [0026] *<br>* paragraph [0038] - paragraph [0040] * | 1-16 | INV.<br>G06F19/00 |
| X | US 2009/281980 A1 (TANIIKE YUKO [JP] ET AL) 12 November 2009 (2009-11-12)<br>* claims 1-3; figures 3,5 * | 1-16 | |
| A | WO 2011/122203 A1 (OMRON HEALTHCARE CO LTD [JP]; OTSUBO YUTAKA [JP]; FUJISAKI AKIYOSHI [J) 6 October 2011 (2011-10-06)<br>* abstract * | 1-16 | |
| A | US 2008/154643 A1 (LEON MAURICIO A [US]) 26 June 2008 (2008-06-26)<br>* paragraph [0031] - paragraph [0033] *<br>* paragraph [0109] - paragraph [0112] * | 1-16 | |
| | | | TECHNICAL FIELDS SEARCHED (IPC)<br><br>G06F |

The present search report has been drawn up for all claims

| Place of search | Date of completion of the search | Examiner |
|---|---|---|
| Munich | 4 May 2012 | Filloy García, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 11 19 1630

This annex lists the patent family members relating to the patent documents cited in the above-mentioned European search report.
The members are as contained in the European Patent Office EDP file on
The European Patent Office is in no way liable for these particulars which are merely given for the purpose of information.

04-05-2012

| Patent document cited in search report | | Publication date | Patent family member(s) | | Publication date |
|---|---|---|---|---|---|
| US 2011125680 | A1 | 26-05-2011 | NONE | | |
| US 2009281980 | A1 | 12-11-2009 | CN | 101341504 A | 07-01-2009 |
| | | | US | 2009281980 A1 | 12-11-2009 |
| | | | WO | 2007072816 A1 | 28-06-2007 |
| WO 2011122203 | A1 | 06-10-2011 | JP | 2011209871 A | 20-10-2011 |
| | | | WO | 2011122203 A1 | 06-10-2011 |
| US 2008154643 | A1 | 26-06-2008 | NONE | | |

EPO FORM P0459

For more details about this annex : see Official Journal of the European Patent Office, No. 12/82